(19) Europäisches Patentamt
European Patent Office
Office européen des brevets

(11) Veröffentlichungsnummer : **0 621 266 A1**

(12) **EUROPÄISCHE PATENTANMELDUNG**

(21) Anmeldenummer : **94810207.4**

(22) Anmeldetag : **13.04.94**

(51) Int. Cl.$^5$ : **C07C 309/51,** C07D 251/68, D06M 13/256

(30) Priorität : **22.04.93 CH 1228/93**

(43) Veröffentlichungstag der Anmeldung :
**26.10.94 Patentblatt 94/43**

(84) Benannte Vertragsstaaten :
**BE CH DE ES FR GB IT LI**

(71) Anmelder : **CIBA-GEIGY AG**
**Klybeckstrasse 141**
**CH-4002 Basel (CH)**

(72) Erfinder : **Bacher, Jean-Pierre**
**rue des Vergers 9**
**F-68220 Buschwiller (FR)**
Erfinder : **Rembold, Manfred, Dr.**
**Im Aeschfeld 21**
**CH-4147 Aesch (CH)**
Erfinder : **Reinehr, Dieter, Dr.**
**Wolfsheule 10**
**D-79400 Kandern (DE)**

(54) **Sterisch gehinderte Phenole und ihre Verwendung zur Stabilisierung von Polyamid-Fasermaterialen.**

(57) Es werden sterisch gehinderte Phenole der Formel

(1) 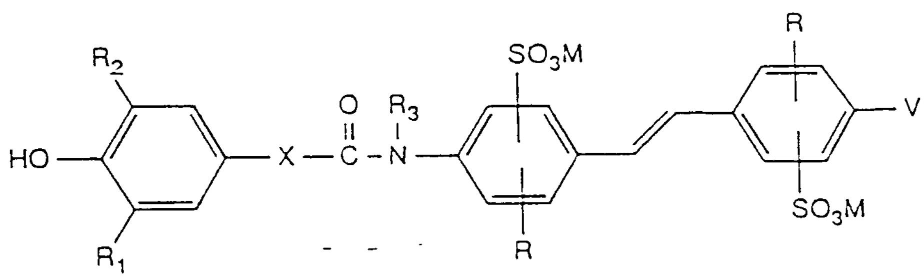 ,

worin
V ein Rest der Formel

(1a) 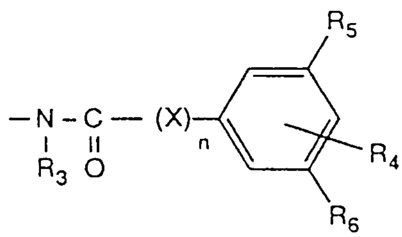 oder (1b) 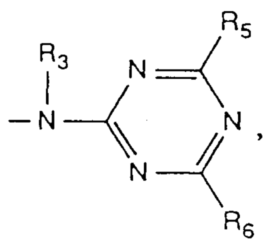 ,

bedeutet ;
wobei M, n, R, $R_1$, $R_2$, $R_3$, $R_4$, $R_5$, $R_6$ und X die in Anspruch 1 angegebene Bedeutung haben, beschrieben. Diese Antioxidantien eignen sich dazu, die thermische und photochemische Stabilität von ungefärbten und gefärbten Polyamid-Fasermaterialien zu erhöhen. Sie zeichnen sich durch eine nahezu pH-unabhängige hohe Ausziehrate aus.

EP 0 621 266 A1

Jouve, 18, rue Saint-Denis, 75001 PARIS

Die vorliegende Erfindung betrifft wasserlösliche, sterisch gehinderte Phenole, ein Verfahren zur Herstellung dieser Verbindungen, ein Verfahren zur photochemischen und thermischen Stabilisierung von Polyamid-Fasermaterialien mit Hilfe dieser Verbindungen sowie das mit den erfindungsgemässen Verbindungen behandelte Fasermaterial.

Die wasserlöslichen, sterisch gehinderten Phenole entsprechen der Formel

(1) 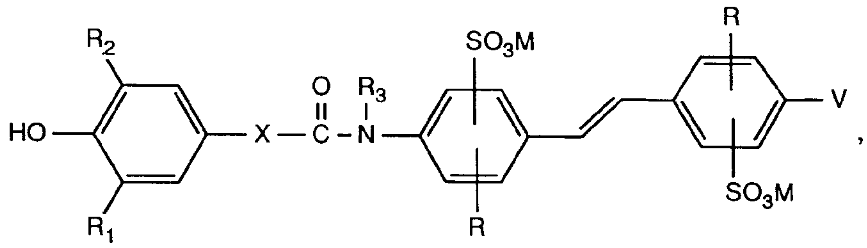 ,

worin

| | |
|---|---|
| R | Wasserstoff, Halogen, Niederalkyl oder Niederalkoxy, |
| $R_1$ und $R_2$, | unabhängig voneinander, Wasserstoff, Methyl, tert.Butyl und die Summe der Kohlenstoffatome von $R_1$ und $R_2$ mindestens 2 beträgt, |
| $R_3$ | Wasserstoff oder eine gegebenenfalls substituierte Alkylgruppe, |
| X | Alkylen, Oxaalkylen oder Thiaalkylen, |
| V | einen Rest der Formel |

(1a) 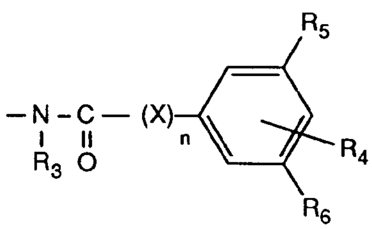 oder (1b) 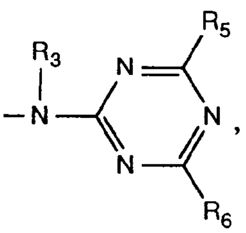 ,

| | |
|---|---|
| $R_4$ | Wasserstoff, Halogen, Niederalkyl oder Niederalkoxy, |
| $R_5$ und $R_6$ | unabhängig voneinander Wasserstoff, Halogen, Niederalkyl, Niederalkoxy, Amino, Mononiederalkylamino, Diniederalkylamino, Phenoxy, Phenylamino oder Phenylniederalkylamino, |
| M | Wasserstoff, Alkalimetall, Erdalkalimetall, Ammonium oder einen organischen Ammoniumrest, und |
| n | 0 oder 1 bedeutet. |

Bei der Definition der Reste R, $R_4$, $R_5$ und $R_6$ stellen Niederalkyl, Niederalkoxy, Mononiederalkylamino und Diniederalkylamino solche Gruppen oder Gruppenbestandteile dar, die 1 bis 5, insbesondere 1 bis 3 Kohlenstoffatome aufweisen. Beispiele für derartige Gruppen sind Methyl, Ethyl, n-Propyl, Isopropyl, n-Butyl, sek.Butyl, tert. Butyl, Amyl oder Isoamyl bzw. Methoxy, Ethoxy, Isopropoxy, Isobutoxy, tert.-Butoxy oder tert.-Amyloxy.

Phenylniederalkylamino bedeutet beispielsweise Phenethyl-, Phenylpropyl-, Phenylbutyl- oder vorzugsweise Benzylamino.

Mononiederalkylamino und Diniederalkylamino können durch Halogen, Niederalkoxy, Hydroxy, Carboxy oder Carboxyniederalkyl substituiert sein. Niederalkoxy kann durch Niederalkoxy substituiert sein. Phenyl kann durch Niederalkyl, Niederalkoxy oder Halogen substituiert sein.

$R_3$ kann als geradkettige oder verzweigte Alkylgruppe 1 bis 18, vorzugsweise 1 bis 8 Kohlenstoffatome enthalten. Beispiele dafür sind die Methyl-, Ethyl-, Isopropyl, Pentyl-, Octyl-, Dodecyl- und Octadecylgruppe.

Als substituierte Alkylgruppe bedeutet $R_3$ beispielsweise eine Hydroxyalkyl-, Alkoxyalkyl, Aminoalkyl-, Alkylaminoalkyl- oder Dialkylaminoalkylgruppe mit insgesamt 2 bis 10, vorzugsweise 2 bis 5 Kohlenstoffatomen. Beispiele dafür sind die β-Hydroxyethyl-, β-Methyoxyethyl-, β-Aminoethyl-, β,β-Diethylaminoethyl- oder die β-Butylaminoethylgruppe.

Halogen bei den Resten R, $R_4$, $R_5$ und $R_6$ bedeutet Fluor, Brom oder vorzugsweise Chlor.

X kann geradkettig oder verzweigt sein und 1 bis 8, vorzugsweise 1 bis 5 Kohlenstoffatome enthalten.

Beispiele dafür sind der Methylen-, Ethylen-, Trimethylen-, Propylen-, 2-Thia-trimethylen- oder 2-Oxapentamethlenrest.

Als Beispiele für Alkalimetalle seien Lithium, Natrium oder Kalium genannt.Beispiele für Erdalkalimetalle sind Calcium und Magnesium. Wegen der Schwerlöslichkeit gewisser Calcium-, Strontium- und Bariumsalze in wasserhaltigen Medien sowie aus ökonomischen Gründen sind dabei Verbindungen der Formel (1) bevorzugt, die in Form ihrer Natrium-, Lithium-, Magnesium- oder Ammoniumsalze oder als Ammoniumsalz einer organischen Sticksoffbase vorliegen. Beispiele für solche Stickstoffbasen, die mit der $SO_3$-Gruppe Ammoniumsalze bilden können, sind Trimethylamin, Triethylamin, Triethanolamin, Diethanolamin, Ethanolamin, Cyclohexylamin, Dicyclohexylamin, Hexamethylenimin oder Morpholin.

Praktisch wichtige sterisch gehinderte Phenole sind solche Verbindungen der Formel (1), bei denen V einen Rest der Formel (1b) oder

(1c) 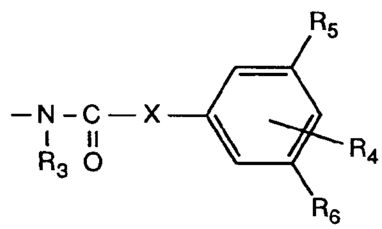

bedeutet und

$R_3$, $R_4$ $R_5$ und X die in Formel (1a) angegebene Bedeutung haben.

Besonders bevorzugt sind Verbindungen, bei denen $R_3$ Wasserstoff oder geradkettiges oder verzweigtes $C_1$-$C_8$-Alkyl, und ganz besonders solche Verbindungen, bei denen $R_3$ Wasserstoff bedeutet.

Unter den Verbindungen der Formel (1) sind diejenigen bevorzugt, bei denen $R_1$ und $R_2$ unabhängig voneinander Methyl oder tert.Butyl bedeuten und ganz besonders solche Verbindungen, bei denen $R_1$ = $R_2$ = tert.Butyl bedeutet.

Im Vordergrund des Interesses stehen Verbindungen der Formel (2)

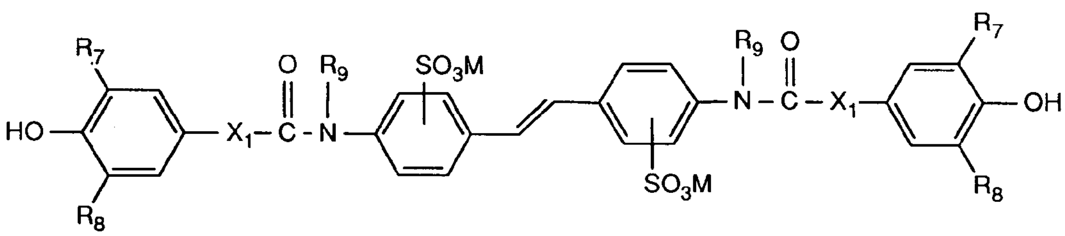,

worin

| | |
|---|---|
| $R_7$ und $R_8$, | unabhängig voneinander Methyl oder tert.Butyl, |
| $R_9$ | Wasserstoff oder Niederalkyl |
| $X_1$ | Niederalkylen und |
| M | Wasserstoff oder Natrium bedeuten. |

Die wasserlöslichen sterisch gehinderten Phenole der Formeln (1) und (2) stellen neue Verbindungen dar. Sie können nach an sich bekannten Methoden hergestellt werden, wie beispielsweise in der US-A-3,665,031 beschrieben. Die Herstellung erfolgt z.B. dadurch, dass man 1 Mol der Verbindung der Formel

(3) 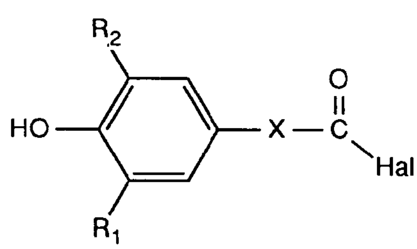

mit 1 Mol der Verbindung der Formel

(4) 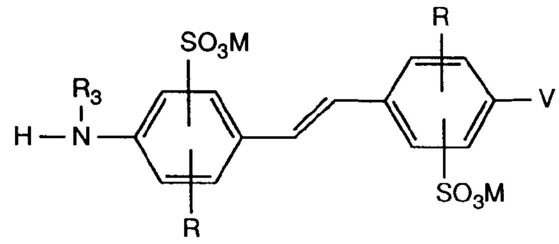

umsetzt, worin

Hal Halogen bedeutet und

R, $R_1$, $R_2$, $R_3$, V, X und M die angegebene Bedeutung haben.

Die wasserlöslichen sterisch gehinderten Phenole eignen sich zur photochemischen und thermischen Stabilisierung von gefärbten und ungefärbten Polyamid-Fasermaterialien.

Aus der US-A-3,665,031 ist bekannt, ungefärbte Polymere, z.B. Polyamide gegen den Einfluss von Hitze und/oder Sauerstoff (Luftoxidation) mit Hilfe von wasserlöslichen phenolischen Antioxidantien zu schützen.

Die US-A-5,096,456 beschreibt ein Verfahren zur Verbesserung der thermischen und/oder photochemischen Stabilität von Färbungen auf Polyamidfasern durch Behandlung der gefärbten Polyamidfasern mit phenolischen wasserlöslichen Antioxidantien.

Gegenüber den dort beschriebenen Antioxidantien zeichnen sich die ausgewählten, erfindungsgemässen Verbindungen durch höhere Ausziehgrade aus, die im wesentlichen pH-unabhängig sind. Dadurch eignen sie sich insbesondere für den Einsatz in Färbebädern, die Metallkomplexfarbstoffe enthalten und einen pH-Wert von 7 bis 7,5 aufweisen.

Das Verfahren zur photochemischen Stabilisierung von Polyamid-Fasermaterialien mit Hilfe der erfindungsgemässen sterisch gehinderten Phenole bildet daher einen weiteren Gegenstand der vorliegenden Erfindung.

Das Verfahren ist dadurch gekennzeichnet, dass man das gefärbte oder ungefärbte Fasermaterial mit einem sterisch gehinderten Phenol der Formel

(1) 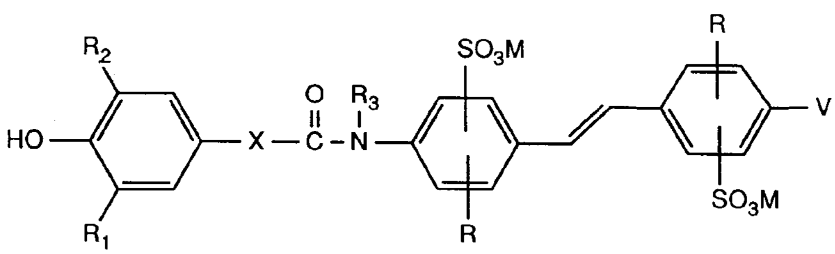

behandelt, worin

| | |
|---|---|
| R | Wasserstoff, Halogen, Niederalkyl oder Niederalkoxy, |
| $R_1$ und $R_2$, | unabhängig voneinander, Wasserstoff, Methyl, tert.Butyl und die Summe der Kohlenstoffatome von $R_1$ und $R_2$ mindestens 2 beträgt |
| $R_3$ | Wasserstoff oder eine gegebenenfalls substituierte Alkylgruppe, |
| X | Alkylen, Oxaalkylen oder Thiaalkylen |
| V | einen Rest der Formel |

(1a) 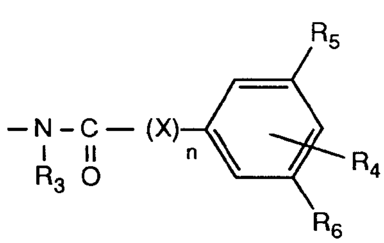 oder (1b) 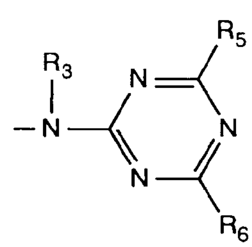

| | |
|---|---|
| $R_4$ | Wasserstoff, Halogen, Niederalkyl oder Niederalkoxy, |
| $R_5$ und $R_6$ | unabhängig voneinander Wasserstoff, Halogen, Niederalkyl, Niederalkoxy, Amino, Mononiederalkylamino, Diniederalkylamino, Phenoxy, Phenylamino oder Phenylniederalkylamino, |
| M | Wasserstoff, Alkalimetall, Erdalkalimetall, Ammonium oder einen organischen Ammoniumrest, und |
| n | 0 oder 1 bedeutet. |

Die Verbindungen der Formel (1 ) können aus üblichen Flotten nach gängigen Methoden auf die Polyamid-Fasermaterialien aufgebracht werden. Erfindungsgemäss werden sie aus wässrigem Bad appliziert, das die Verbindungen in einer Menge von 0,005 bis 10 Gew.-%, vorzugsweise 0,05 bis 2 Gew.-% enthält. Vorzugsweise wird die Behandlung mit den erfindungsgemässen Verbindungen und die Färbung im gleichen Applikationsbad vorgenommen. Man kann dabei so vorgehen, dass dem wässrigen Applikationsbad zuerst das Antioxidans zugefügt, das entsprechende Fasermaterial behandelt und anschliessend die Färbung durchgeführt wird, oder man kann gleichzeitig dem Bad das Antioxidans und den Farbstoff zugeben oder zunächst die Färbung durchführen und dann die Behandlung mit dem das Antioxidans vornehmen. Die gleichzeitige Applikation von Antioxidans und Farbstoff ist bevorzugt. Die Applikation kann dabei nach einem Auszieh- oder Kontinueverfahren erfolgen.

Beim Ausziehverfahren kann das Flottenverhältnis innerhalb eines weiten Bereiches gewählt werden, z.B. 1:5 bis 1:300, vorzugsweise 1:10 bis 1:50. Man arbeitet zweckmäßig bei einer Temperatur von 30 bis 120°C, vorzugsweise 50 bis 98°C.

Beim Kontinue-Verfahren beträgt der Flottenauftrag zweckmäßig 30-400 Gew.-%, vorzugsweise 75-250 Gew.-%. Zur Fixierung der applizierten Farbstoffe und der erfindungsgemässen Verbindungen wird das Fasermaterial einer Hitzebehandlung unterworfen. Der Fixierprozess kann auch nach der Kaltverweilmethode erfolgen.

Die Hitzebehandlung erfolgt vorzugsweise durch ein Dämpfverfahren unter Behandlung in einem Dämpfer mit gegebenenfalls überhitztem Dampf bei einer Temperatur von 98 bis 105°C während z.B. 1 bis 7, vorzugsweise 1 bis 5 Minuten. Die Fixierung der Farbstoffe und der Verbindungen der Formel (1) gemäss dem Kaltverweilverfahren kann durch Lagerung der imprägnierten und vorzugsweise aufgerollten Ware bei Raumtemperatur (15 bis 30°C), z.B. während 3 bis 24 Stunden erfolgen, wobei die Kaltverweilzeit bekanntlich von der Art des applizierten Farbstoffes abhängig ist.

Nach Beendigung des Färbeprozesses bzw. der Fixierung werden die hergestellten Färbungen auf übliche Weise gespült und getrocknet.

Man erhält mit dem erfindungsgemässen Verfahren Polyamid-Färbungen und -fasern mit guter thermischer und photochemischer Stabilität.

Als die erfindungsgemäss zu stabilisierenden Färbungen kommen solche in Betracht, die durch Säure- oder Metallkomplexfarbstoffe, z.B. 1:2-Chrom, 1:2-Kobaltkomplexfarbstoffe oder Kupferkomplexfarbstoffe oder Dispersions- und Reaktivfarbstoffe erzeugt werden.

Beispiele für solche Farbstoffe sind in Colour Index, 3. Auflage, 1971, Band 4, beschrieben.

Unter Polyamidfasermaterial wird solches aus synthetischem Polyamid, wie z.B. Polyamid 6, Polyamid 6,6 oder Polyamid 12, sowie modifiziertes Polyamid, z.B. basisch anfärbbares Polyamid verstanden. Neben den reinen Polyamidfasern kommen vor allem auch Fasermischungen aus Polyurethan und Polyamid in Betracht, so z.B. Trikotmaterial aus Polyamid/Polyurethan im Mischungsverhältnis 70:30. Grundsätzlich kann das reine oder gemischte Polyamidfasermaterial in den verschiedensten Verarbeitungsformen vorliegen, wie z.B. als Faser, Garn, Gewebe, Gewirke, Vlies oder Flormaterial.

Das vorliegende Verfahren eignet sich besonders vorteilhaft zur Behandlung von Polyamidfasermaterial, das Licht und Hitze ausgesetzt wird und z.B. als Autopolsterstoff oder Teppich Verwendung findet.

Die folgenden Beispiele veranschaulichen die Erfindung. Teile bedeuten Gewichtsteile und Prozente Gewichtsprozente.

Beispiel 1:

18,4 g 4,4,-Diaminostilben-2,2'-disulfonsäure werden zusammen mit 37 g Tributylamin in 400 ml Dimethylformamid bei 70°C gelöst. Es wird auf 50°C abgekühlt, mit 81 g einer toluolischen Lösung von β-(4-Hydroxy-3,5-di.tert.butyl-phenyl)-propionsäurechlorid (55%ig) versetzt und während 24 Stunden bei 50°C gerührt. Die Reaktionslösung wird am Wasserstrahlvakuum eingedampft und der Rückstand in 500 ml Essigester verrührt, abfiltriert und mit weiteren 500 ml Essigester gewaschen. Man erhält 44 g eines hellbeigen Produktes der Formel

(101) 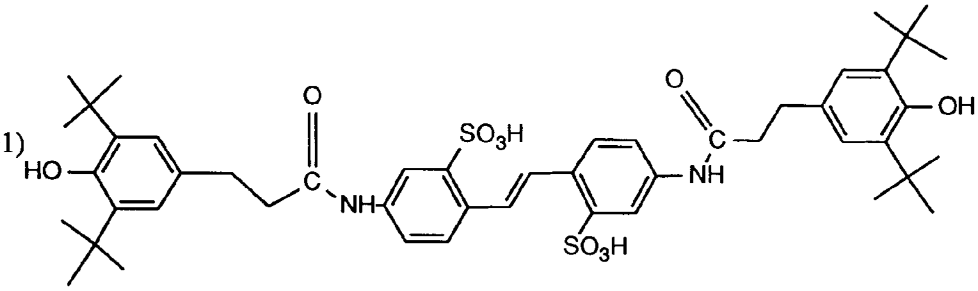

Ausbeute: 94% d.Th.
$\lambda_{max}$ = 337 nm

Beispiel 2:

Verwendet man anstelle von 4,4'-Diaminostilben-2,2'-disulfonsäure die folgende Aminostilbenverbindung der Formel

(102a) 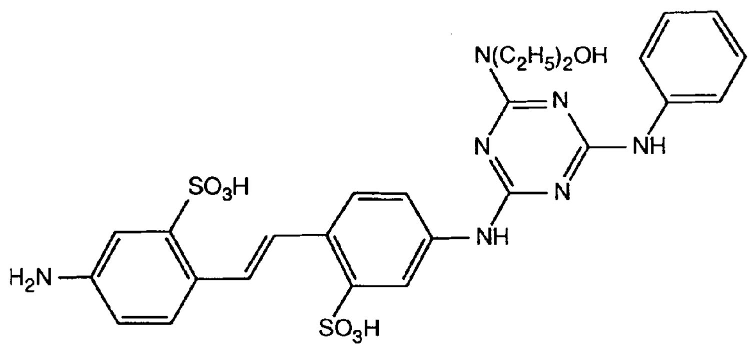 ,

so erhält man cie Verbindung der Formel (102)

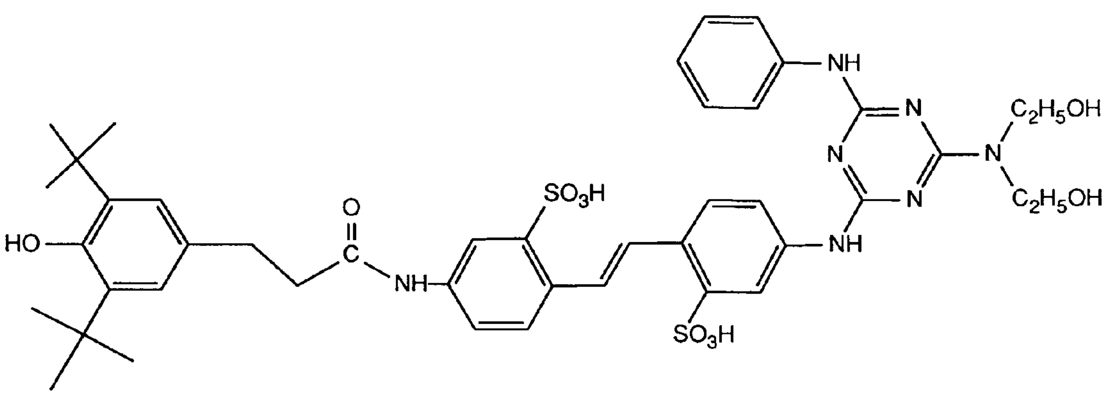

in ähnlicher Ausbeute.

Die Verbindung der Formel (102a) wird durch Umsetzung von 4-Amino-4'-nitro-2,2'-disulfonsäure mit Cyanurchlorid, anschliessender Reaktion mit Anilin und Diethylamin und abschliessender Bechamp-Reduktion der Nitro- zu einer Aminogruppe hergestellt.

Beispiele 3 bis 6:

Analog zu Beispiel 2 lassen sich die folgenden Verbindungen herstellen:

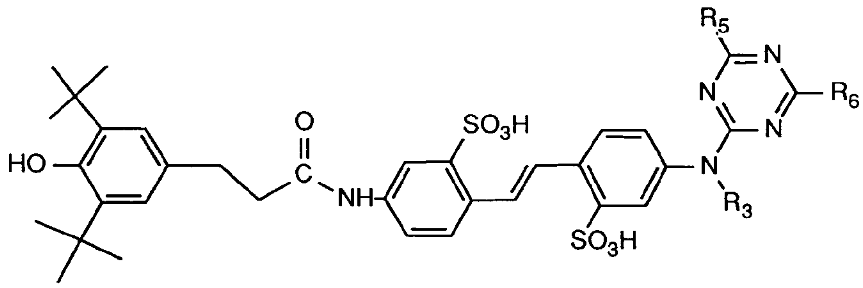

Tabelle 1:

| Verbindung Nr. | $R_3$ | $R_5$ | $R_6$ |
|---|---|---|---|
| (103) | H | 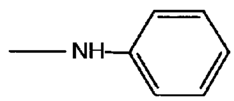 | Morpholino |
| (104) | H | 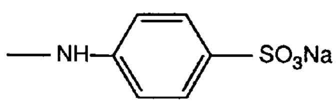 | 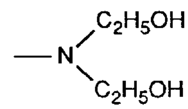 |
| (105) | H | 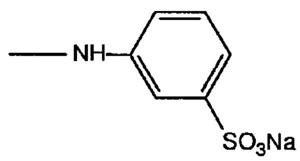 | 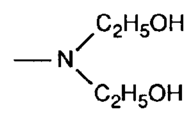 |
| (106) | H | 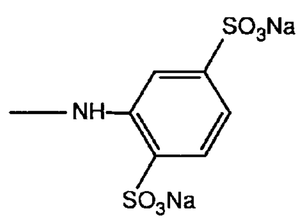 | Morpholino |

Anwendungsbeispiel

Beisspiel 2:

2 Muster von je 10 g einer PA 6 Maschenware (Lilion Texturtricot 5-4003) werden in einem Färbeapparat, z.B. Vistacolor der Firma Zeller bei einem Flottenverhältnis von 1:25 gefärbt. Die 2 Bäder enthalten noch folgende Zusätze:

1 g/l Na-Phosphat im Verhältnis 25 Teile Mono-Na zu 175 Teilen Di-Na Salz (pH 7,5), sowie

1%, bezogen auf das textile Material, eines nichtionischen Egalisierhilfsmittels

Während die Flotte 1 keine weiteren Zusätze enthält, wird der Flotte 2 jeweils noch 1%, bezogen auf das textile Material, der Verbindung der Formel (101) zugegeben.

In die so vorbereiteten Flotten geht man bei 45°C ein und erhitzt innerhalb von 45 Minuten auf 95°C. Nach einer Färbezeit von 40 Minuten bei 95°C kühlt man auf 60°C ab, spült mit kaltem Wasser, zentrifugiert und

trocknet das Gewebe bei Raumtemperatur.

Die so behandelten Muster werden entsprechend DIN 75.202 (FAKRA) belichtet und ihre Reissfestigkeiten nach SN 198.461 gemessen. Die Ergebnisse sind in Tabelle 2 dargestellt:

Tabelle 2:

| Zusatz zum Färbebad | Reissfestigkeit nach 120 h FAKRA Belichtung in % |
|---|---|
| kein Zusatz | 10 |
| 1 % der Verbindung (101) | 31 |

2 weitere Muster werden einem Hitzetest unterzogen. Die Muster werden dabei für 72 Stunden in einem Umluftofen bei 140°C gealtert und anschliessend der Reisswert nach SN 198.461 bestimmt. Die Ergebnisse sind in Tabelle 3 dargestellt:

Tabelle 3:

| Zusatz zum Färbebad | Reissfestigkeit nach 72 h bei 140°C in % |
|---|---|
| kein Zusatz | 10 |
| 1% der Verbindung (101) Ausziehgrad 94% | 91 |

Das mit der erfindungsgemässen Verbindung behandelte Gewirke ergibt sowohl bei photochemischer als auch thermischer Beanspruchung deutlich höhere Reissfestigkeiten.

## Patentansprüche

**1.** Sterisch gehinderte Phenole der Formel

(1) 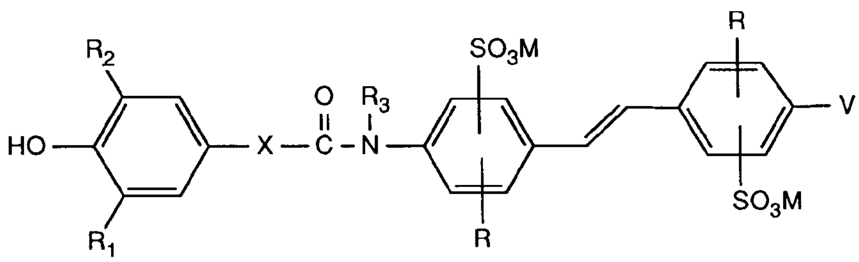

worin

| | |
|---|---|
| R | Wasserstoff, Halogen, Niederalkyl oder Niederalkoxy, |
| $R_1$ und $R_2$, | unabhängig voneinander, Wasserstoff, Methyl, tert.Butyl und die Summe der Kohlenstoffatome von $R_1$ und $R_2$ mindestens 2 beträgt |
| $R_3$ | Wasserstoff oder eine gegebenenfalls substituierte Alkylgruppe, |
| X | Alkylen, Oxaalkylen oder Thiaalkylen |
| V | einen Rest der Formel |

(1a) 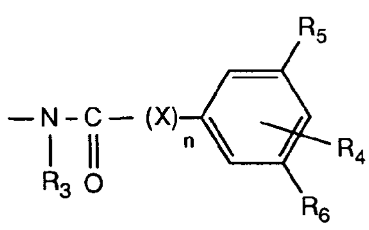 oder (1b) 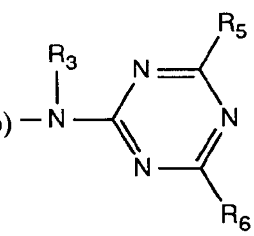

| | |
|---|---|
| $R_4$ | Wasserstoff, Halogen, Niederalkyl oder Niederalkoxy |
| $R_5$und $R_6$ | unabhängig voneinander Wasserstoff, Halogen, Niederalkyl, Niederalkoxy, Amino, Mononiederalkylamino, Diniederalkylamino, Phenoxy, Phenylamino oder Phenylniederalkylamino, |
| M | Wasserstoff, Alkalimetall, Erdalkalimetall, Ammonium oder einen organischen Ammoniumrest, und |
| n | 0 oder 1 bedeutet. |

2. Sterisch gehindertes Phenol der Formel (1) nach Anspruch 1 worin V einen Rest der Formel (1b) oder (1c)

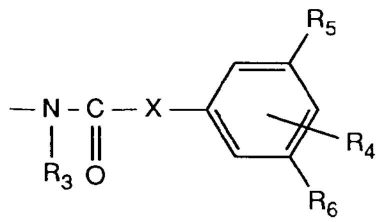

bedeutet, wobei
$R_3$, $R_4$, $R_5$, $R_5$ und X die in Anspruch 1 angegebene Bedeutung haben.

3. Sterisch gehindertes Phenol nach Anspruch 1 oder 2, dadurch gekennzeichnet, dass X ein geradkettiges oder verzweigtes $C_1$-$C_8$-Alkylen ist.

4. Sterisch gehindertes Phenol nach einem der Ansprüche 1 bis 3, dadurch gekennzeichnet, dass $R_3$ Wasserstoff oder geradkettiges oder verzweigtes $C_1$-$C_8$-Alkyl bedeutet.

5. Sterisch gehindertes Phenol nach einem der Ansprüche 1 bis 4, dadurch gekennzeichnet, dass $R_3$ Wasserstoff bedeutet.

6. Sterisch gehindertes Phenol nach einem der Ansprüche 1 bis 5, dadurch gekennzeichnet, dass $R_1$ und $R_2$ unabhängig voneinander Methyl oder tert.Butyl bedeuten.

7. Sterisch gehindertes Phenol nach Anspruch 1 der Formel (2)

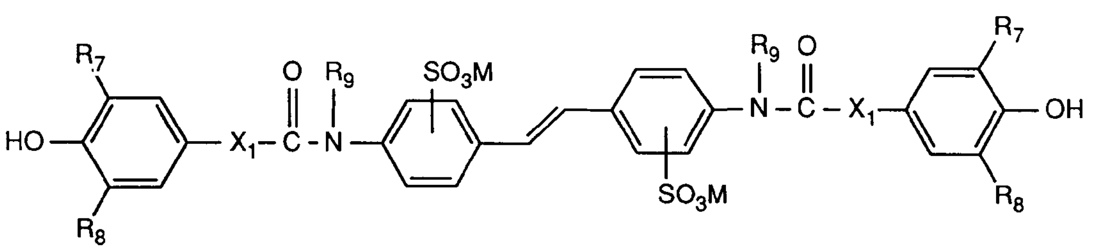

worin

| | |
|---|---|
| $R_7$ und $R_8$, | unabhängig voneinander Methyl oder tert.Butyl, |
| $R_9$ | Wasserstoff oder Niederalkyl |
| $X_1$ | Niederalkylen und |
| M | Wasserstoff oder Natrium bedeuten. |

8. Verfahren zur Herstellung der sterisch gehinderten Phenole der Formel (1), dadurch gekennzeichnet, dass man 1 Mol der Verbindung der Formel

(3) 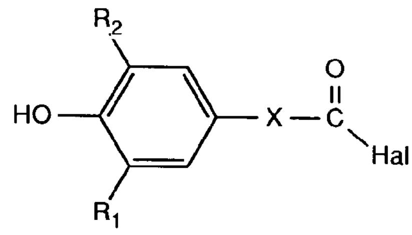

mit 1 Mol der Verbindung der Formel

(4) 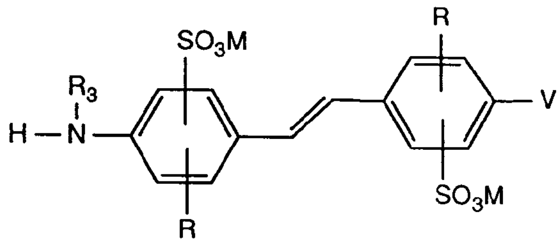

umsetzt, worin
Hal Halogen bedeutet und
R, $R_1$, $R_2$, $R_3$, V, X und M die in Anspruch 1 angegebene Bedeutung haben.

**9.** Verfahren zur photochemischen und thermischen Stabilisierung von gefärbten und ungefärbten Polyamid-Fasermaterialien, dadurch gekennzeichnet, dass man das gefärbte oder ungefärbte Fasermaterial mit einem sterisch gehinderten Phenol der Formel

(1) 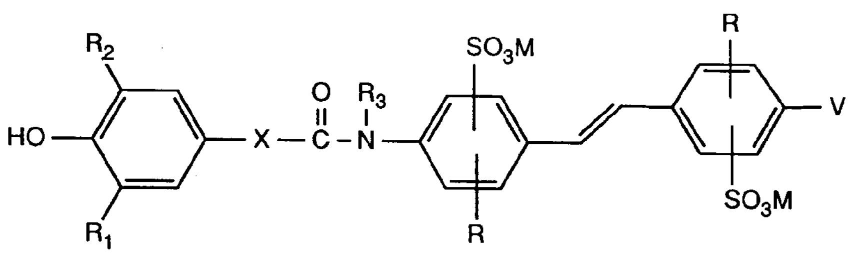

behandelt, worin
R Wasserstoff, Halogen, Niederalkyl oder Niederalkoxy,
$R_1$ und $R_2$, unabhängig voneinander, Wasserstoff, Methyl, tert.Butyl und die Summe der Kohlenstoffatome von $R_1$ und $R_2$ mindestens 2 beträgt
$R_3$ Wasserstoff oder eine gegebenenfalls substituierte Alkylgruppe,
X Alkylen, Oxaalkylen oder Thiaalkylen
V einen Rest der Formel

(1a) 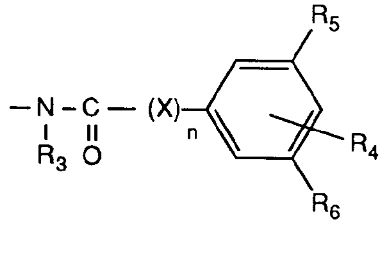

oder

(1b) 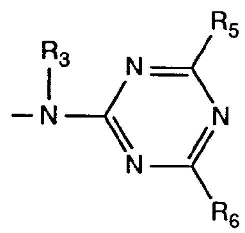

| | |
|---|---|
| $R_4$ | Wasserstoff, Halogen, Niederalkyl oder Niederalkoxy |
| $R_5$ und $R_6$ | unabhängig voneinander Wasserstoff, Halogen, Niederalkyl, Niederalkoxy, Amino, Mononiederalkylamino, Diniederalkylamino, Phenoxy, Phenylamino oder Phenylniederalkylamino, |
| M | Wasserstoff, Alkalimetall, Erdalkalimetall, Ammonium oder einen organischen Ammoniumrest, und |
| n | 0 oder 1 bedeutet. |

**10.** Das mit dem sterisch gehinderten Phenol der Formel (1) behandelte Fasermaterial.

Europäisches Patentamt

# EUROPÄISCHER RECHERCHENBERICHT

Nummer der Anmeldung
EP 94 81 0207

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| A<br><br><br>D | EP-A-0 438 381 (CIBA-GEIGY)<br>* das ganze Dokument, insbesondere Verbindung 34 *<br>& US-A-5 096 456 | 1,9 | C07C309/51<br>C07D251/68<br>D06M13/256 |
| A | EP-A-0 459 950 (CIBA-GEIGY)<br>* das ganze Dokument, insbesondere Verbindung 34 * | 1,9 | |
| A | EP-A-0 475 907 (CIBA-GEIGY)<br>* das ganze Dokument * | 1,9 | |
| A | CHEMICAL ABSTRACTS, vol. 76, no. 12, 20. März 1972, Columbus, Ohio, US;<br>abstract no. 60909g,<br>Y. YAMASHITA: 'Fluorescent whitening agents. III. Fluorescence and photostability of aqueous solutions of N-substituted derivatives of sodium 4,4'-diamino-2-2'-stilbenedisulphonate'<br>Seite 66-67 ;<br>& YUKI GOSEI KAGAKU KYOAKAI SHI, 1971, 29(5), 519-525 | 1 | **RECHERCHIERTE SACHGEBIETE (Int.Cl.5)**<br>C07C<br>C07D |

Der vorliegende Recherchenbericht wurde für alle Patentansprüche erstellt

| Recherchenort | Abschlußdatum der Recherche | Prüfer |
|---|---|---|
| DEN HAAG | 7. Juni 1994 | English, R |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)